# EUROPÄISCHE PATENTSCHRIFT

(11) **EP 2 027 841 B1**
(45) Veröffentlichungstag und Bekanntmachung des Hinweises auf die Patenterteilung: **11.07.2012**
(21) Anmeldenummer: 08013929.8
(22) Anmeldetag: 04.08.2008
(51) Int. Cl.: A61F 13/15, B32B 5/04, B32B 37/14

(54) **Elastisches Element und Verfahren zum Herstellen einer elastischen Flächenanordnung**
Elastic element and method for producing an elastic area assembly
Élément élastique et procédé de fabrication d'un agencement de surface élastique

(30) Priorität: 03.08.2007 DE 102007036742
(43) Veröffentlichungstag der Anmeldung: 25.02.2009
(60) Teilanmeldung: 11009339.0
(73) Patentinhaber: Koester GmbH & Co. KG, 96146 Altendorf (DE)
(72) Erfinder: Lanz, Jörg, 91052 Erlangen (DE); Neugebauer, Robert, 91077 Neunkirchen (DE)
(74) Vertreter: Reuther, Martin

(56) Entgegenhaltungen:
- EP-A- 1 065 046
- EP-A- 1 273 280
- WO-A-01/87589
- WO-A-02/18125
- WO-A-03/053318
- US-A1- 2005 095 942
- US-A1- 2006 035 055

## Beschreibung

Die Erfindung betrifft ein elastisches Element. Des Weiteren betrifft die Erfindung ein Einweg-Äbsorptionsprodukt sowie eine Windel oder ein Babywindel. Auch betrifft die Erfindung ein Verfahren zum Herstellen einer elastischen Flächenordnung.

So zeigen die DE 10 2005 011 059 B3, die US 6,964,720 B2, die US 6,885,223 B2, die US 2006/0185135 A1 und die EP 1 563 817 A1 derartige elastischen Flächenanordnungen beziehungsweise derartige elastischen Flächenelemente, bei denen elastische Bändchen vorgespannt zwischen zwei Vliesstofflagen eingebracht werden. Auch ist es aus der DE 10 200 011 040 A1 und der US 2006/0246803 A1 bekannt, derartige elastische Flächenanordnungen beziehungsweise derartige elastische Flächenelemente mit elastischen Elementen, die einen gespritzten oder gegossenen Strang umfassen und zwischen Vliesstofflagen eingebettet sind, zu versehen.

Solche elastischen Flächenanordnungen beziehungsweise elastischen Flächenelemente sind beispielsweise auch aus der europäischen Patentschrift EP 1 448 134 B1 bekannt, in welcher ein dehnbares Verbundmaterial beschrieben ist, welches ein streckbares Faser-Trägermaterial, eine Vielzahl von ersten elastomeren Elementstreifen und eine Vielzahl von zweiten elastomeren Elementstreifen aufweist. Diese elastomeren Elemente werden beispielsweise geradlinig auf ein streckbares Substrat aufgebracht, wobei die ersten elastomeren Elemente zueinander beabstandet und die zweiten elastomeren Elemente ebenfalls beabstandet zueinander auf dem streckbaren Substrat positioniert sind. Mittels der elastomeren Elemente ist das dehnbare Verbundmaterial nicht nur dehnbar sondern darüber hinaus auch elastisch.

In einem diesbezüglichen ersten Ausführungsbeispiel sind die beiden unterschiedlichen elastomeren Elemente hinsichtlich ihrer Erstreckungsrichtung rechtwinklig zueinander angeordnet, wodurch ein damit ausgestattetes streckbares Substrat entsprechend in zwei rechtwinklig zueinander angeordnete Richtungen jeweils eine gewünschte Dehneigenschaft erhält.

Um hiervon hinsichtlich eines alternativen Verbundmaterials abweichende Dehneigenschaften zu erzielen, können die beiden unterschiedlichen elastomeren Elemente abweichend von einem rechten Winkel miteinander verbunden auf einem streckbaren Substrat angebracht werden.

Durch die beiden unterschiedlich zueinander ausgerichteten elastomeren Elementstreifen können einem streckbaren Substrat je nach gewählter Erstreckungsrichtung der elastomeren Elementstreifen unterschiedliche Dehneigenschaften zuteil werden.

Mittels der in der EP 1 448 134 B1 beschriebenen streckbaren Verbundmaterialen mit ihren zueinander beabstandeten elastomeren Elementstreifen ist es möglich, auf die Verwendung von großflächig und zusammenhängend ausgebildeten elastomeren Elementen zu verzichten und somit einen nicht unbeträchtlichen Anteil an elastomeren Material einzusparen. Darüber hinaus lassen sich auf Grund der variablen Dehneigenschaften insbesondere in einer Hauptdehnrichtung des streckbaren Verbundmaterials eine verbesserte Passform und ein verbesserter Tragekomfort erzielen.

Weiter ist in der internationalen Patentanmeldung WO 01/87589 A2 eine Vorrichtung beschrieben, mittels welcher zwischen zwei dehnbaren Gewebetlächen zum einen geradlinige elastische Endlosfilamente und zum anderen geradlinige Klebstofflinien, mittels welchen die erste Gewebefläche und die zweite Gewebefläche miteinander verklebt werden, eingebracht werden. Mittels der linienförmig aufgetragenen Endlosfilamente erhalten die dehnbaren Gewebeflächen zusätzlich eine Elastizität unter Einsparung von elastischem und großflächig zusammenhängendem Material, welches in der Anschaffung und der Herstellung relativ teuer ist.

Ähnlich verhält es sich hinsichtlich der Absorptionartikel, welche in der US 2005/0215972 A1 bzw. der WO 2005/097029 A1 beschrieben sind, und bei welchen etliche Bereiche der Absorptionsartikel mit Dehnstreifen oder mit Dehnzonen ausgestattet sind. Insbesondere werden mittels der Dehnstreifen Windelohren von Babywindeln elastisch gestaltet, indem derartige Dehnstreifen auf ein Trägermaterial aufgebracht werden. Jedoch sind die gezeigten Dehnstreifen, insbesondere die gezeigten Dehnzonen, relativ teuer herzustellen, da sie ein relativ großes Materialvolumen, beispielsweise an thermoplastischen Elastomeren, erfordern.

Aus ist es aus der WO 2003/053318 bekannt. Höschenwindeln mit elastischen Beinbünden zu versehen, die einen oder zwei mäanderförmige elastische Stränge umfassen, die miteinander verbunden sind.

Es ist Aufgabe vorliegender Erfindung gattungsgemäße Einweg-Absorptionsprodukte mit elastischen Bereichen auszustatten, welche gleiche oder verbesserte Dehneigenschaften aufweisen, die kostengünstiger bereitgestellt werden können.

Die Aufgabe der Erfindung wird von einem elastischen Element mit den Merkmalen des Anspruchs 1 gelöst.

Insbesondere kann eine elastische Flächenanordnung mit einem elastischen Element vorgesehen sein, das wenigstens einen mäanderförmig ausgebildeten Strang umfasst, der thermisch mit einer Trägerbahn verbunden ist.

In der Praxis hat sich überraschenderweise herausgestellt, dass insbesondere mäanderförmig ausgebildete Stränge wesentlich bessere Dehneigenschaften hervorbringen als beispielsweise geradlinig ausgeformte elastische Filamente, da Bereiche des Strangs durch die Mäanderform des Strangs bedingt auch quer zu einer Hauptdehnrichtung verlaufen, so dass in diesem Bereich, also in Hauptdehnrichtung gesehen, eine größere Anzahl an molekular vernetztem Material angehäuft werden kann, ohne entlang einer in Hauptdehnrichtung vorgesehenen Gesamtstrecke einen breiten thermoplastischen Elastomerstreifen über die Gesamtstrecke vorsehen zu müssen.

Hierdurch gelingt es, entweder gleiche Dehneigenschaften einer dehnbaren Trägerbahn bei weniger Verbrauch an thermoplastischem Elastomer zu verwirklichen oder verbesserte Dehneigenschaften der so ausgestatteten dehnbaren Trägerbahn zu realisieren.

Insbesondere können auf diese Weise sowohl senkrecht als auch parallel zu einer Maschinenrichtung Stränge aus elastischem Material gelegt werden, so dass ohne weiteres auch senkrecht zur Maschinenrichtung eine Elastizität gewährleistet werden kann.

Durch ein thermisches Verbinden des Stranges mit der Trägerbahn verbindet sich der Strang zudem besonders innig mit der Trägerbahn, so dass ein unbeabsichtigtes Ablösen des Strangs selbst bei großer Zugbelastung von der Trägerbahn verringert ist, und kann schnell und betriebssicher aufgebracht werden.

Als elastische Flächenanordnung ist vorteilhafter Weise jeder Bereich beispielsweise eines Einweg-Absorptionsproduktes kostengünstig herstellbar, welcher mit einem solchen elastischen Element versehen werden soll oder kann.

Mit dem Begriff "Strang" wird vorliegend ein Gebilde aus einem gespritzten oder gegossenen elastischem Material beschrieben, wie etwa ein thermoplastisches Elastomer. Der hier erfingdungsgemäß verwendete Strang zeichnet sich zudem durch eine in sich geschlossene Oberfläche aus. Das heißt, dem Strang innewohnende Molekülketten sind an der Strangobertläche im Gegensatz zu geschnittenen beziehungsweise gestanzten Materialsträngen nicht durchtrennt, sondern "unverletzt" und somit auf molekularer Ebene vernetzt miteinander verbunden.

Ein solcher Strang ist besonders einfach herzustellen, wenn der Strang gespritzt beziehungsweise gegossen ist.

Die "Trägerbahn" kann vorliegend vielfältig realisiert sein, wobei beispielsweise Strickstoffe, gewebte Faserbahnen oder Fliesfaserbahnen, aber auch Folien zur Anwendung kommen können. Bevorzugt ist die Trägerbahn dehnbar ausgelegt, so dass die elastischen Eigenschaften des elastischen Strangs sich vorteilhaft auf die Trägerbahn auswirken können. Beispielsweise handelt es sich bei einer Trägerbahn um dehnbare Vliesbahnen aus Polyoletin-Fasern oder -filamenten, wie etwa Polyethylen oder Polypropylen. Ebenso kann die Dehnbarkeit auch durch ein nachträgliches Aktivieren bereitgestellt werden, bei welchem die strukturelle Integrität der Trägerbahn und/oder der Verbindung zwischen Trägerbahn und Strang teilweise zerstört wird.

Besonders vorteilhaft ist es, wenn der verwendete Strang ein Querschnittsverhältnis Höhe/Breite von über 1/5, vorzugsweise von über 1/2, aufweist. Bevorzugt weist der Strang ein Querschnittsverhältnis von 1 zu 1 auf, wodurch er besonders schmal und schlank baut und sich hierdurch besonders materialsparend gegenüber aus dem Stand der Technik bekannten und relativ breiten Dehnstreifen abhebt. Somit lassen sich selbst größere Bereiche, wie beispielsweise komplette Windelohren, elastisch dehnbar ausbilden, ohne hierbei den Kostenrahmen zu sprengen.

Ist der Strang vollflächig mit der Trägerbahn verbunden, ist die elastische Flächenanordnung besonders belastbar und hält eine besonders hohe Anzahl an Belastungszyklen aus.

Des Weiteren wird die Aufgabe der Erfindung von einem elastischen Element, umfassend zumindest drei Stränge aus TPE gelöst, die jeweils mäanderförmig ausgebildet sind und von denen wenigstens ein erster Strang mit den beiden anderen Strängen unmittelbar an wenigstens zwei diskreten Verbindungsstellen verbunden ist. Die Stränge aus einem thermoplastischen Elastomer lassen sich auf der Trägerbahn besonders gut netzartig miteinander verbinden, so dass jeder Strang nicht unabhängig und allein für sich gesehen auf die Trägerbahn sondern vorteilhafter Weise in einem zusammenhängenden Verbund auf die Trägerbahn wirkt.

Konstruktiv besonders einfach lassen sich zwei Stränge zu einer Verbindungsstelle miteinander verbinden, wenn an wenigstens einer Verbindungsstelle zwei Stränge über einen Klebstoffpunkt miteinander verbunden sind.

Eine solche Verbindung lässt sich alternativ besonders einfach realisieren, wenn die Stränge in der Verbindungsstelle miteinander verschmelzen. Hierdurch sind die Stränge auf molekularer Ebene besonders innig miteinander verbunden.

Eine bevorzugte Ausführungsvariante sieht vor, dass an wenigstens einer Verbindungsstelle zwei Stränge miteinander verschweißt, vorzugsweise ultraschallverschweißt oder laserverschweißt, sind. Vorteilhafterweise ergibt sich durch eine derart gestaltete Verbindungsstelle, dass diese lediglich eine einfache Stärke eines Einzelstrangs aufweist, so dass das elastische Element selbst im Bereich der Verbindungsstellen in einer im Wesentlichen konstanten Stärke beziehungsweise Dicke vorliegt.

Eine solche konstante Stärke über das gesamte elastische Element kann ebenfalls erreicht werden, wenn an wenigstens einer Verbindungsstelle zwei Stränge im flüssigen bzw. plastisch verförmbaren Zustand miteinander verpresst sind.

Gegebenenfalls erfolgt an der Verbindungsstelle sogar eine chemische Verbindung der Stränge, wenn diese in flüssigen bzw. plastisch verformbaren und somit wenigstens teilweise reaktiven Zustand verpresst werden.

Insbesondere ein derart hergestelltes elastisches Element ist vorteilhaft, wenn es auf einer Trägerbahn angeordnet ist, da es in einem Arbeitsschritt ebenfalls auf die Trägerbahn aufgepresst werden kann.

Eine Trägerbahn erfährt zum einen besonders gute Dehneigenschaften und ist zum anderen besonders hochwertig mit dem elastischen Element verbunden, wenn das elastische Element flächig mit der Trägerbahn verbunden ist. Wie bereits vorstehend erwähnt, gehen die Stränge des elastischen Elementes eine besonders innige Verbindung mit der Trägerbahn ein, wenn das elastische Element flächig mit der Trägerbahn verbunden ist.

Eine solche innige Verbindung ist relativ leicht herzustellen, wenn das elastische Element thermisch mit der Trägerbahn verbunden ist.

Die Aufgabe der Erfindung wird darüber hinaus einerseits von einem elastischen Flächenelement gelöst, welches sich durch Maschen auszeichnet, die derart ausgerichtet sind, dass die Elastizität des Flächenelements in Maschinenrichtung kleiner sind als in Querrichtung.

Vorliegend können Maschen des elastischen Flächenelementes dadurch, bereitgestellt werden, dass wenigstens zwei Stränge wenigstens zwei diskrete Verbindungsstellen miteinander aufweisen. Auch können diese Maschen durch Ausschnitte, Ausstanzungen oder Bedrucken und ähnliches erzeugt werden.

Mit dem Begriff "Maschinenrichtung" wird die Richtung bezeichnet, in welcher sich das elastische Flächenelement beziehungsweise die Trägerbahn während des Herstellungsprozesses durch die Maschine bewegt. Die Maschinenrichtung fällt hiermit also mit der Transportrichtung der Trägerbahn beziehungsweise des elastischen Flächenelementes durch die Maschine zusammen, mittels welcher das elastische Flächenelement hergestellt wird. Die Maschinenrichtung erstreckt sich des Weiteren vorzugsweise senkrecht zur vorstehend beschriebenen Hauptdehnrichtung des elastischen Elementes.

Demzufolge erstreckt sich die "Querrichtung" ebenfalls senkrecht zur Maschinenrichtung. Das elastische Flächenelement wird demnach während seines Einsatzes vorzugsweise in Querrichtung gedehnt. Somit fallen die Querrichtung des elastischen Flächenelementes und die Hauptdehnrichtung des elastischen Flächenelementes im Wesentlichen zusammen.

Die Asymmetrie der Elastizität lässt sich beispielsweise ohne weiteres durch eine entsprechende Asymmetrie der Maschen bzw. deren Umrandung gewährleisten.

Da die Elastizität des Flächenelements in Maschinenrichtung kleiner sind als in Querrichtung, besitzt das elastische Flächenelement in seiner Hauptdehnrichtung straffere Dehneigenschaften als in Maschinenrichtung, wodurch vorteilhafter Weise ein erforderlicher Mindestwiderstand senkrecht zur Hauptdehnrichtung mit einem kostengünstig herzustellenden netzartig ausgebildeten elastischen Flächenelement erzielt wird.

Andererseits wird die Ausgabe der Erfindung von einem elastischen Flächenelement mit Maschen, deren Begrenzung aus zumindest zwei Maschinenrichtungsbegrenzungen und zumindest zwei Querrichtungsbegrenzungen gebildet ist, wobei die Maschinenrichtungsbegrenzung in Maschinenrichtung im Mittel von der Maschinenrichtung mehr abweicht als die Querrichtungsbegrenzung in Querrichtung im Mittel von der Querrichtung abweicht.

Der Begriff "Maschinenrichtungsbegrenzung" beschreibt einen Teilbereich eines Stranges oder mehrerer Strängen aus einem thermoplastischen Elastomer, der bzw. die sich in Maschinenrichtung erstrecken und eine Masche zwischen zwei im Wesentlichen nebeneinander liegenden Verbindungsstellen begrenzen. Dementsprechend beschreibt der Begriff "Querrichtungsbegrenzung" einen Teilbereich eines Stranges oder mehrerer Stränge aus einem thermoplastischen Elastomer, der bzw. die sich in Querrichtung erstrecken und eine Masche ebenfalls zwischen zwei im Wesentlichen nebeneinander liegenden Verbindungsstellen begrenzen. Insoweit ist zu berücksichtigen, dass eine "Maschinenrichtungsbegrenzung" zwar im Wesentlichen in Maschinenrichtung ausgerichtet ist, eine Masche jedoch letztlich im Wesentlichen in Querrichtung begrenzt, während eine "Querrichtungsbegrenzung" zwar im Wesentlichen in Querrichtung ausgerichtet ist, die Masche jedoch im Wesentlichen in Maschinenrichtung begrenzt.

Vorliegend erfährt ein elastisches Element in seiner Hauptdehnrichtung besonders brauchbare Dehneigenschaften, wenn eben die Maschinenrichtungsbegrenzung in Maschinenrichtung im Mittel von der Maschinenrichtung mehr abweicht als die Querrichtungsbegrenzung in Querrichtung im Mittel von der Querrichtung abweicht.

Hierdurch ist festgelegt, dass die Teilbereiche des Stranges, welche sich im Wesentlichen in Querrichtung erstrecken weniger kurvig, beispielsweise auf einer Trägerbahn, angeordnet sind als die Teilbereiche des Stranges, die sich im Wesentlichen in Maschinenrichtung erstrecken. Hierdurch erhält das elastische Flächenelement in seiner Hauptdehnrichtung bessere Dehneigenschaften als in einer hiervon abweichenden Dehnrichtung.

Des Weiteren wird die Aufgabe der Erfindung von einem elastischen Flächenelement mit Maschen mit einer Maschinenerstreckung und einer Quererstreckung gelöst, wobei die Maschinenerstreckung kleiner als die Quererstreckung ist. Hierdurch ist das elastische Flächenelement elastischer in Quererstreckung ausgebildet als in der Maschinenerstreckung, so dass ein derart ausgebildetes elastisches Flächenelement in seiner Hauptdehnrichtung besonders brauchbare Dehneigenschaften aufweist.

Auch wird die Aufgabe der Erfindung von einem elastischen Flächenelement mit Maschen gelöst, deren elastische Umgrenzung in eine Maschinenrichtung eine höhere Materialdichte als in eine Querrichtung hierzu aufweist. Durch diese Ausgestaltung wird das elastische Flächenelement in Maschinenrichtung stabilisiert, dass heißt, dass in dieser Richtung auftretende Kräfte zu geringeren Ausdehnungen führen, als dieses bei senkrecht hierzu ausgerichteten Kräften der Fall ist. In diesem Zusammenhang versteht es sich, dass der Vergleich der Materialdichten zumindest im Mittel über die Größe einer Masche zu erfolgen hat, um zu einer diesbezüglich relevanten Aussage zu gelangen, wobei jedoch auch größere Gebiete des elastischen Flächenelements, insbesondere mehrere Maschen, richtungsabhängig entsprechend verglichen werden können. Eine derartige Asymmetrie kann durch eine geeignete Aktivierung, die lediglich in Querrichtung durchgerührt wird, noch ergänzt werden, wobei es sich versteht, dass eine derartige asymmetrische Aktivierung auch unabhängig hiervon bei den vorliegenden dargestellten Lösungen zur Anwendungen kommen kann.

Die Maschen lassen sich baulich besonders einfach herstellen, wenn die Maschen durch miteinander verbundene Stränge gebildet werden.

Die Stränge lassen sich auf der Trägerbahn besonders einfach auftragen, wenn die Stränge in Maschinenrichtung fortschreiten.

Das elastische Flächenelement erhält eine gute Steifigkeit, wenn das elastische Flächenelement auf einer Trägerbahn angeordnet ist. Beispielsweise ist das elastische Flächenelement auf einem streckbaren Gewebe aufgetragen, welches - je nach konkreter Ausführungsform - eigenstabil ausgebildet sein oder seiner Stabilität erst durch die Verbindung mit dem elastischen Flächenelement erhalten kann.

Es versteht sich, dass die Trägerbahn durch eine Vielzahl an Materialien gebildet sein kann. Eine bevorzugte Ausführungsvariante insbesondere hinsichtlich Einweg-Absorptionsprodukte sieht in diesem Zusammenhang vor, dass die Trägerbahn ein Non-Woven umfasst. NonWoven weist eine textile Haptik aus und damit ist für einen Anwender besonders angenehm.

Es versteht sich, dass die vorstehend erläuterte Flächenanordnung, das vorstehend erläuterte elastische Element sowie das vorstehend erläuterte elastische Flächenelement vielseitig einsetzbar sind. Besonders vorteilhaft ist es jedoch, diese im Bereich von Einweg-Absorptionsprodukten einzusetzen, da hier einerseits hochwertig ausgebildete Produkte verwendet werden müssen, um einen besonders guten Tragekomfort zu gewährleisten, insbesondere wenn die Einweg-Absorptionsprodukte für Babys bestimmt sind. Andererseits sind diese vorteilhaft, da mit ihnen selbst große Bereiche eines Einweg-Absorptionsproduktes kostengünstig ausgestattet werden können und hierdurch elastische Eigenschaften erhalten.

Deshalb sieht eine weitere Lösung ein Einweg-Absorptionsprodukt, wie eine Windel bzw. eine Babywindel, vor, weiches sich durch eine elastische Flächenanordnung, ein elastisches Element und/oder ein elastisches Flächenelement mit den vorstehend erläuterten Merkmalen auszeichnet.

Gelöst wird die Aufgabe auch von einem Verfahren zum Herstellen einer elastischen Flächenanordnung, insbesondere eines Einweg-Absorptionsproduktes, wie einer Windel, bei welchem ein thermoplastisches Elastomer in Kontakt mit einer Trägerbahn gebracht und das thermoplastische Elastomer als Kurven auf die Trägerbahn aufgebracht wird.

Ein als Kurven auf eine Trägerbahn aufgebrachtes Elastomer verleiht der Trägerbahn bei geringem Materialverbrauch hinsichtlich des thermoplastischen Elastomers sehr gute und gegenüber Geraden verbesserte elastische Dehneigenschaften, da sich der Elastomer nicht nur eindimensional sondern zweidimensional auf der Trägerbahn erstreckt. Hierdurch steht, wie vorstehend bereits beschrieben, in einigen Bereichen mehr Elastomermaterial zur Verfügung als in anderen, wodurch sich die Dehneigenschaften insbesondere in der Hauptdehnrichtung verbessern.

Die Kurven sind vorzugsweise als sinusförmig geschwungene Stränge aus einem thermoplastischen Elastomer gebildet. Somit sind sie einfach realisierbar. Vorzugsweise werden die Kurven auf die Trägerbahn aufgespritzt oder aufgegossen.

Vorzugsweise werden die Kurven in Maschinenrichtung im Wesentlichen kontinuierlich aufgebracht. Eine derartige Vorgehensweise ermöglicht einen äußerst sparsamen und gezielten Materialeinsatz und reduziert mithin die Kosten. Dementsprechend ist es vorteilhaft, wenn die jeweiligen Stränge, welche die Maschen des hierdurch bereitgestellten Flächenelementes bilden, während des Durchtrennens der Materialbahn lediglich an der senkrecht zur Maschinenrichtung verlaufenden Schnittkante durchtrennt werden. Es versteht sich, dass dieses jedoch nicht zwingend ist, wenn beispielsweise die in erfindungsgemäßer Weise bereitgestellte elastische Flächenanordnung mehrnutzig ausgebildet und in Querrichtung nachträglich noch durchtrennt wird.

Um das thermoplastische Elastomer und die Trägerbahn besonders innig miteinander zu verbinden, sieht eine Verfahrensvariante vor, dass das thermoplastische Elastomer mit einer Temperatur oberhalb von 100° C, vorzugsweise oberhalb 150° C, als Kurve auf die Trägerbahn aufgebracht wird.

Verbindungsstellen zwischen zwei Strängen lassen sich verfahrenstechnisch einfach herstellen, wenn erste Kurven eines thermoplastischen Elastomers auf einen ersten Träger und zweite Kurven eines thermoplastischen Elastomers auf einen zweiten Träger aufgebracht und anschließend die beiden Träger zusammen gepresst werden und hierdurch erste Kurven mit zweiten Kurven miteinander verbunden werden. Ebenso können die Kurven übereinander auf eine Trägerbahn aufgebracht und dann die zweite Trägerbahn hierüber gelegt werden. Eine Verbindung mit der Trägerbahn oder einem anderen Träger ist andererseits nicht zwingend notwendig, da die Kurven beispielsweise auch auf einer Kühlwalze aufgelegt, miteinander verbunden und nach dem Erstarren wieder abgenommen werden können. Auch versteht es sich, dass die Kurven nicht zwingen zwischen zwei Trägerbahnen eingebracht werden müssen, sie können auch lediglich auf einer Trägerbahn abgelegt und geeignet mit dieser verbunden sein.

Es versteht sich, dass das thermoplastische Elastomer der ersten Kurven von dem thermoplastischen Elastomer der zweiten Kurven verschieden sein kann, um so die Dehneigenschaften des dehnbaren Produktes zu variieren. Besonders kostengünstig gestaltet sich das Verfahren jedoch, wenn das thermoplastische Elastomer der ersten Kurven und das thermoplastische Elastomer der zweiten Kurven identisch sind.

Eine nahezu einfache Materialstärke in den Verbindungsstellen zweier Kurven wird erzielt, wenn die ersten Kurven mit den zweiten Kurven in Verbindungsstellen miteinander verschmelzen.

Ein Netzmuster beziehungsweise Maschen als elastisches Flächenelement lassen sich besonders einfach realisieren, wenn die ersten Kurven und die zweiten Kurven periodisch gelegt und dann phasenversetzt miteinander verbunden werden.

Vorteilhaft ist es, wenn mittels erster Kurven und zweiter Kurven Maschen mit einer Maschinenerstreckung und einer Quererstreckung gebildet werden, wobei die Maschinenerstreckung kleiner als die Quererstreckung ist. Hierdurch lassen sich insbesondere elastische Flächenelemente mit den vorstehend beschriebenen Vorteilen problemlos herstellen.

Zur Realisierung des vorstehend beschriebenen Herstellungsverfahrens können Kontaktverfahren, wie beispielsweise Tiefdruck, Intaglio-Druck, Flexographiedruck, Spaltbeschichtung, Gießbeschichtung und dergleichen eingesetzt werden. Als kontaktfreie Verfahren sind beispielsweise Farbstrahldrucken, Sprühen oder dergleichen einsetzbar.

Hierbei können die Zusammensetzung, die Temperatur und/oder die Konzentration des thermoplastischen Elastomers variiert werden, um so unterschiedlichsten Ansprüchen gerecht zu werden.

Weitere Vorteile, Ziele und Eigenschaften vorliegender Erfindung werden anhand nachfolgender Erläuterung anliegender Zeichnung beschrieben, in welcher beispielhaft eine Verfahrensanordnung und ein hierdurch hergestelltes elastisches Flächenelement dargestellt sind. Es zeigt
- Figur 1: schematisch eine perspektivische Ansicht einer Anordnung, mittels wel- cher thermoplastisches Elastomer auf Trägerbahnen aufgebracht wird und
- Figur 2: schematisch eine Aufsicht auf ein elastisches Flächenelement mit Ma- schen.

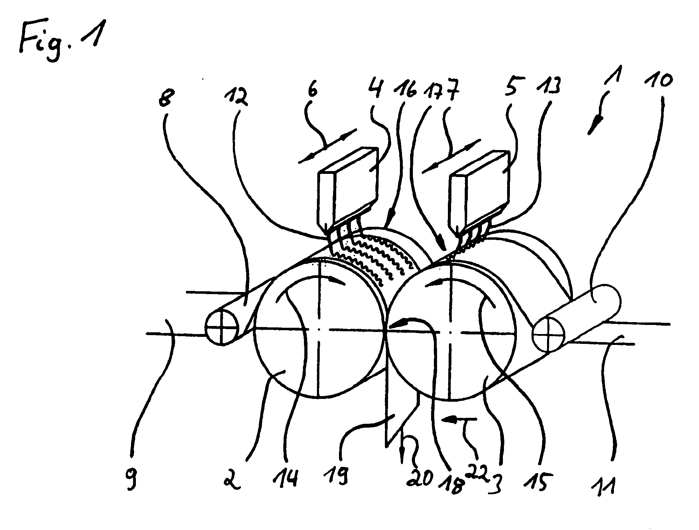

Die in der Figur 1 gezeigte Anordnung 1 umfasst eine erste Anpresswalze 2 und eine zweite Anpresswalze 3, die gegenläufig zueinander berührend rotieren. Hierzu rotiert in der Darstellung nach Figur 1 die erste Anpresswalze 2 im Uhrzeigersinn und die zweite Anpresswalze 3 entgegen des Uhrzeigersinns.

Oberhalb der Anpresswalzen 2 und 3 befinden sich entsprechend eine erste Extrudierbatterie 4 und eine zweite Extrudierbatterie 5. Die beiden Extrudierbatterien 4 und 5 sind gemäß der Doppelpfeile 6 und 7 quer zu den Umlaufrichtungen der ersten Anpresswalze 2 und der zweiten Anpresswalze 3 traversierend angeordnet.

Links neben der ersten Anpresswalze 2 ist eine Zuführwalze 8 vorgesehen, mittels welcher der ersten Anpresswalze 2 eine erste NonWoven-Bahn 9 zugeführt wird. Dementsprechend befindet sich rechts der zweiten Anpresswalze 3 eine zweite Zuführwalze 10, mittels welcher der zweiten Anpresswalze 3 eine zweite NonWoven-Bahn 11 zugeführt wird.

Sowohl die erste Non-Woven-Bahn 9 als auch die zweite Non-Woven-Bahn 11 werden jeweils über den oberen Scheitelpunkt der jeweiligen Anpresswalze 2, 3 geführt und in diesem Bereich mit ersten Strängen 12 (hier nur exemplarisch beziffert) aus der ersten Extrudierbatterie 4 bzw. mit zweiten Strängen 13 (hier nur exemplarisch beziffert) aus der zweiten Extrudierbatterie 5 beaufschlagt. Während die einzelnen Stränge 12. 13 aus entsprechenden Düsen (hier nicht explizit dargestellt) der ersten Extrudierbatterie 4 und der zweiten Extrudierbatterie 5 austreten, bewegen sich die Extrudierbatterien 4 und 5 gemäß der Doppelpfeile 6 und 7 mit zuvor eingestellten Amplituden quer zu den Anpresswalzen 2 und 3. In Verbindung mit der jeweiligen Transportrichtung 14, 15 der beiden NonWoven-Bahnen 9 und 11 ergeben sich auf der ersten NonWoven-Bahn 9 erste Kurven 16 und auf der zweiten NonWoven-Bahn 11 zweite Kurven 17, die jeweils in Maschinenrichtung durchgehend laufen und senkrecht hiezu eine Kurvenbahn beschrieben die um eine Mittellinie schwankt.

Die ersten Stränge 12 und die zweiten Stränge 13 sind phasenversetzt zueinander auf die jeweilige Non-Woven-Bahn 9, 11 aufgegossen, so dass sie im Anpressbereich 18 nicht deckungsgleich miteinander verschmolzen werden.

Im vorliegenden Ausführungsbeispiel bestehen die Stränge 12 und 13 aus einem thermoplastischen Elastomer (TPE), welches im Anpressbereich 18 immer noch als Schmelze vorliegt, so dass einerseits die Stränge 12 und 13 mit den Non-Woven-Bahnen 9 und 11 eine innige Verbindung eingehen und andererseits die ersten Stränge 12 und die zweiten Stränge 13 untereinander verschmelzen, sofern sie sich gegenseitig berühren.

Mittels der ersten Stränge 12 und der zweiten Stränge 13, die zumindest im Anpressbereich 18 noch als Schmelze beziehungsweise angeschmolzen vorliegen, werden die beiden NonWoven-Bahnen 9 und 11 auch dauerhaft miteinander "verbunden". Vorteilhafterweise kann hierbei auf die Klebkraft eines zusätzlichen Klebers, mittels welchen die beiden NonWoven-Bahnen 9 und 11 verklebt werden, verzichtet werden, was jedoch nicht zwingend ist.

Die so mittels der Stränge 12 und 13 miteinander verbundenen NonWoven-Bahnen 9 und 11 verlassen nach dem Anpressbereich 18 als elastisches Flächenelement 19 die Anordnung 1 in Maschinenrichtung 20, wobei das elastische Flächenelement 19 nun zur Weiterverarbeitung, etwa an Babywindeln zur Verfügung, steht.

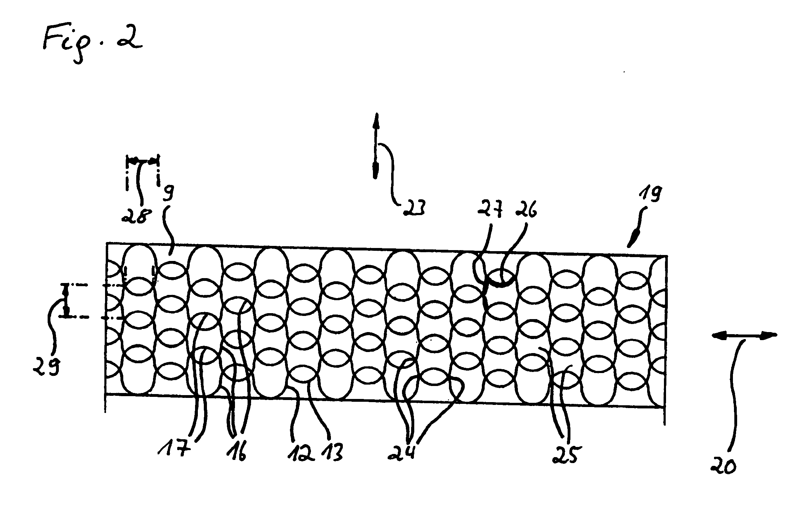

In der Aufsicht nach der Figur 2 ist das elastische Flächenelement 19 aus Blickrichtung 22 (siehe Figur 1) ohne die zweite NonWoven-Bahn 11 dargestellt, um so einen Einblick auf die miteinander vernetzten ersten Kurven 16 und zweiten Kurven 17, welche aus den ersten Strängen 12 und den zweiten Strängen 13 gebildet sind, zu erhalten.

Die Kurven 16 liegen in Querrichtung 23 gesehen nebeneinander beabstandet auf der ersten NonWoven-Bahn 9 angeordnet. Insgesamt befinden sich bei diesem Ausführungsbeispiel vier erste Stränge 12 nebeneinander, in Querrichtung gesehen, angeordnet auf der ersten Non-Woven-Bahn 9. Des Weiteren sind weiter vier zweite Stränge 13 nebeneinander, in Querrichtung 23 gesehen, versetzt über den Strängen 12 angeordnet.

Alle Stränge 12, 13 haben denselben Kurvenverlauf, jedoch sind die ersten Stränge 12 gegenüber den zweiten Strängen 13 phasenversetzt auf der ersten Non-Woven-Bahn 9 angeordnet, so dass die ersten Stränge 12 und die zweiten Stränge 13 sich in Verbindungsstellen 24 schneiden. Die Stränge 12, 13 sind an den Verbindungsstellen miteinander verschmolzen, da die Stränge 12, 13 als aufgeschmolzenes thermoplastisches Elastomer auf die Non-Woven-Bahnen 9, 11 aufgetragen werden und zum Zeitpunkt des Zusammenfügens zumindest noch in einem angeschmolzenen Zustand vorliegen, so dass sich die Stränge 12 und 13 auf molekularer Ebene miteinander verbinden, wodurch sehr stabile Verbindungsstellen 24 geschaffen werden.

Besonders vorteilhaft ist ein derartiges Verschmelzen, da die beiden Stränge 12, 13 in den Verbindungsstellen 24 keine größeren oder zumindest keine wesentliche vergrößerten Querschnitte aufweisen als in Querschnittsbereichen der Stränge 12, 13 an nicht Verbindungsstellen.

Die so miteinander verschmolzenen Stränge 12, 13 beziehungsweise Kurven 16, 17 bilden bereits ohne die beiden Non-Woven-Bahnen 9 und 11 ein elastisches Element aus einer Vielzahl von Maschen 25. Eine Masche ist hierbei zumindest mittels einer Maschinenrichtungsbegrenzung 26 (exemplarisch verdickt dargestellt) und zumindest einer Querrichtungsbegrenzung 27 (exemplarisch verdickt dargestellt), wobei die Querrichtungsbegrenzung eine Verbindungsstelle einschließt, gebildet. Hierbei weicht die Maschinenrichtungsbegrenzung 26 in Maschinenrichtung 20 im Mittel von der Maschinenrichtung 20 mehr ab als die Querrichtungsbegrenzung 27 in Querrichtung 23 im Mittel von der Querrichtung 23 abweicht. Vorteilhafter Weise ergibt sich hierdurch, dass die Elastizität des durch die Maschen 25 gebildeten elastischen Flächenelements 19 in Maschinenrichtung 20 kleiner sind als in Querrichtung 23. Dies hat den Vorteil, das das fertige elastische Flächenelement 19 in Querrichtung 23, also in seiner Hauptdehnrichtung beziehungsweise in seiner Arbeitsrichtung elastischer ist als in Maschinenrichtung 20. Dies ist auch darauf zurückzuführen, dass die Maschinenrichtungsbegrenzung 26 eine Maschinenerstreckung 28 aufweist, welche kleiner ist als eine Quererstreckung 29 einer Querrichtungsbegrenzung 27 des aus Maschen 25 gebildeten elastischen Flächenelementes 19.

Wie unmittelbar aus Figur 2 ersichtlich, weist das elastische Flächenelement schon aus rein geometrischen Gründen in seinen Maschinenrichtungsbegrenzungen 26 der Maschen 25 eine wesentlich höhere Materialdichte auf als in seinen Querrichtungsbegrenzungen 27. Auf diese Weise ist das elastische Flächenelement 19 in Maschinenrichtung stabilisiert, so dass eine Applikation an einer Windel oder einem sonstigen Gegenstand sowie ein Handling der entsprechenden Materialbahn erheblich erleichtert ist.

Da sich die jeweiligen Kurven aufgrund einer kontinuierlichen Fördergeschwindigkeit bzw. Drehgeschwindigkeit der Anpresswalzen 2, 3 sowie der travisierenden Bewegung der Extrudierbatterien 4, 5 in ihrer Materialdicke ändern, da die Relativgeschwindigkeiten der Extrudierbatterien 4, 5 einerseits und der Anpresswalzen 2, 3 andererseits entsprechend variieren, so dass darüber hinaus auch die Dicken der Stränge 12, 13 bzw. Kurven 16, 17 entsprechend variieren und in Maschinenrichtungen 20 stärker ausgebildet sind als in Querrichtung 23. Hierdurch wird das elastische Flächenelement 19 über die eigentliche Anordnung der Kurven hinaus entsprechend mit einer asymmetrischen Materialdichte ausgebildet, die dieses in Maschinenrichtung 20 noch weiter stabilisiert. Durch einen mehrdimensionalen Bewegungsablauf der Extrudierbatterien 4, 5 lässt sich die Dicke darüber hinaus noch anpassen bzw. vergleichmäßigen. Selbiges gilt durch eine entsprechende Geschwindigkeitsvariation der Extrudierbatterien.

Bei vorliegendem Ausführungsbeispiel haben die Stränge 12, 13 beim Austritt der Extrudierbatterien 4, 5 einen im Wesentlichen kreisförmigen Querschnitt. Sie werden jedoch zwischen den Anpresswalzen 2, 3 allmählich platt gedrückt, so dass jedoch das Höhen-Breitenverhältnis einen Wert von 1/3 nicht wesentlich unterschreitet. Hierbei wird die Breite der Stränge 12, 13 in einer durch die Maschinenrichtung 20 und die Querrichtung 23 aufgespannten Ebene gemessen, während die Höhe der Stränge 12, 13 senkrecht zu dieser Ebene gemessen wird.

### Bezugszeichenliste:

- 1: Anordnung
- 2: erste Anpresswalze
- 3: zweite Anpresswalze
- 4: erste Extrudierbatterie
- 5: zweite Extrudierbatterie
- 6: erster Doppelpfeil
- 7: zweiter Doppelpfeil
- 8: erste Zuführwalze
- 9: erste Non-Woven-Bahn
- 10: zweite Zuführwalze
- 11: zweite Non-Woven-Bahn
- 12: erste Stränge
- 13: zweite Stränge
- 14: erste Transportrichtung
- 15: zweite Transportrichtung
- 16: erste Kurven
- 17: zweite Kurven
- 18: Anpressbereich
- 19: elastisches Flächenelement
- 20: Maschinenrichtung
- 22: Blickrichtung
- 23: Querrichtung
- 24: Verbindungsstellen
- 25: Maschen
- 26: Maschinenrichtungsbegrenzung
- 27: Querrichtungsbegrenzung
- 28: Maschinenerstreckung
- 29: Quererstreckung

## Patentansprüche

1. Elastisches Element, umfassend zumindest drei Stränge aus TPE, die jeweils mäanderförmig ausgebildet sind und von denen wenigstens ein erster Strang (12) mit den beiden anderen Strängen (13) unmittelbar an wenigstens zwei diskreten Verbindungsstellen (24) verbunden ist.

2. Elastisches Element nach Anspruch 1, ***dadurch gekennzeichnet, dass*** an wenigstens einer Verbindungsstelle (24) zwei der Stränge (12, 13) über einen Klebstoffpunkt miteinander verbunden sind.

3. Elastisches Element nach Anspruch 1 oder 2, ***dadurch gekennzeichnet, dass*** an wenigstens einer Verbindungsstelle (24) zwei der Stränge (12, 13) miteinander verschweißt, vorzugsweise ultraschallverschweißt oder laserverschweißt, sind.

4. Elastisches Element nach einem der Ansprüche 1 bis 3, ***dadurch gekennzeichnet, dass*** an wenigstens einer Verbindungsstelle (24) zwei der Stränge (12, 13) in flüssigem bzw. plastisch verformbarem Zustand miteinander verpresst sind.

5. Elastisches Element nach einem der vorstehenden Ansprüche, **dadurch gekennzeichnet, dass** das wenigstens einen mäanderförmig ausgebildeten Strang umfasst, der thermisch mit einer Trägerbahn verbunden ist.

6. Elastisches Element nach Anspruch 5, ***dadurch gekennzeichnet, dass*** der Strang (12, 13) gespritzt oder gegossen ist.

7. Elastisches Element nach Anspruch 5 oder 6, ***gekennzeichnet durch*** einen Strang (12, 13) mit einem Querschnittsverhältnis Höhe/Breite von über 1/5, vorzugsweise von über 1/2.

8. Einweg-Absorptionsprodukt, ***gekennzeichnet durch*** ein elastisches Element nach einem der vorstehenden Ansprüche.

9. Windel oder Babywindel, ***gekennzeichnet durch*** ein Windelohr oder einen Wiridelvenchluss mit einem elastischen Element nach einem der vorstehenden Ansprüche.

10. Verfahren zum Herstellen einer elastischen Flächenanordnung bei welchem ein thermoplastisches Elastomer in Kontakt mit einer Trägerbahn gebracht wird, ***dadurch gekennzeichnet, dass*** das thermoplastische Elastomer als Kurven (16, 17) auf die Trägerbahn (9, 11) aufgebracht wird, wobei erste Kurven und zweite Kurven periodisch gelegt und phasenversetzt miteinander verbunden werden.

11. Herstellverfahren nach Anspruch 10, ***dadurch gekennzeichnet, dass*** das thermoplastische Elastomer mit einer Temperatur oberhalb 100° C, vorzugsweise oberhalb 150° C, als Kurven (16, 17) auf die Trägerbahn (9. 11) aufgebracht wird.

12. Herstellverfahren nach Anspruch 10 oder 11, ***dadurch gekennzeichnet, dass*** erste Kurven (16) eines thermoplastischen Elastomers auf einen ersten Träger (9) und zweiten Kurven (17) eines thermoplastischen Elastomers auf einen zweiten Träger (11) aufgebracht und anschließend die beiden Träger (9, 11) zusammengepresst werden und hierdurch die ersten Kurven (16) mit den zweiten Kurven (17) miteinander verbunden werden.

## Claims

1. An elastic element, comprising at least three strands of TPE, each of which is designed in a meandering shape, and of which at least one first strand (12) is directly connected with the other two strands (13) at at least two discrete connecting locations (24).

2. The elastic element in accordance with Claim 1, **characterised in that** at at least one connecting location (24) two of the strands (12, 13) are connected with one another by means of an adhesive dot.

3. The elastic element in accordance with Claim 1 or 2, **characterised in that** at at least one connecting location (24) two of the strands (12, 13) are welded together, are preferably ultrasound-welded or laser-welded.

4. The elastic element in accordance with one of the Claims 1 to 3, **characterised in that** at at least one connecting location (24) two of the strands (12, 13) are pressed together in a fluid state, or in a plastically deformable state.

5. The elastic element in accordance with one of the preceding claims, **characterised in that** it comprises at least one strand designed in a meandering shape, which is thermally connected with a supporting web.

6. The elastic element in accordance with Claim 5, **characterised in that** the strand (12, 13) is injected or cast.

7. The elastic element in accordance with Claim 5 or 6, **characterised by** a strand (12, 13) with a height/width cross-sectional ratio of more than 1/5, preferably of more than 1/2.

8. A one-way absorption product, **characterised by** an elastic element in accordance with one of the preceding claims.

9. A nappy or a baby nappy, **characterised by** a nappy tab or a nappy fastener with an elastic element in accordance with one of the preceding claims.

10. A method for the manufacture of a planar elastic arrangement, in which a thermoplastic elastomer is brought into contact with a supporting web, **characterised in that** the thermoplastic elastomer is applied as curves (16, 17) onto the supporting web (9, 11), wherein first curves and second curves are laid periodically and connected with one another in a phase-displaced manner.

11. The manufacturing method in accordance with Claim 10, **characterised in that** the thermoplastic elastomer is applied at a temperature above 100°C, preferably above 150°C, as curves (16, 17) onto the supporting web (9, 11).

12. The manufacturing method in accordance with Claim 10 or 11, **characterised in that** first curves (16) of a thermoplastic elastomer are applied onto a first supporting structure (9) and second curves (17) of a thermoplastic elastomer are applied onto a second supporting structure (11), and subsequently the two supporting structures (9, 11) are pressed together and by this means the first curves (16) are connected together with the second curves (17).

## Revendications

1. Elément élastique, comprenant au moins trois branches en TPE, lesquelles sont réalisées respectivement en forme de méandre et dont au moins une première branche (12) est reliée aux deux autres branches (13) directement sur au moins deux points de liaison (24) discrets.

2. Elément élastique selon la revendication 1, **caractérisé en ce que** deux des branches (12, 13) sont reliées l'une à l'autre par un point de colle sur au moins un point de liaison (24).

3. Elément élastique selon la revendication 1 ou 2, **caractérisé en ce que** deux des branches (12, 13) sont soudées l'une à l'autre, de préférence soudées par ultrasons ou soudées par laser sur au moins un point de liaison (24).

4. Elément élastique selon l'une des revendications 1 à 3, **caractérisé en ce que** deux des branches (12, 13) sont comprimées l'une avec l'autre dans l'état liquide ou plastiquement déformable sur au moins un point de liaison (24).

5. Elément élastique selon l'une des revendications précédentes, **caractérisé en ce qu'**il comprend au moins une branche conçue en forme de méandre, qui est reliée thermiquement à une bande porteuse.

6. Elément plastique selon la revendication 5, **caractérisé en ce que** la branche (12, 13) est injectée ou coulée.

7. Elément plastique selon la revendication 5 ou 6, **caractérisé par** une branche (12, 13) ayant un rapport de section hauteur/largeur supérieur à 1/5, de préférence supérieur à 1/2.

8. Produit d'absorption à usage unique, **caractérisé par** un élément élastique selon l'une des revendications précédentes.

9. Couche ou couche pour bébé, **caractérisé par** un coin de couche ou une fermeture de couche avec un élément élastique selon l'une des revendications susmentionnées.

10. Procédé pour fabriquer un agencement de surface élastique, dans lequel un élastomère thermoplastique est mis en contact avec une bande support, **caractérisé en ce que** l'élastomère thermoplastique est appliqué sous forme de courbes (16, 17) sur la bande support (9, 11), des premières courbes et des secondes courbes étant placées périodiquement et reliées les unes aux autres de façon déphasée.

11. Procédé de fabrication selon la revendication 10, **caractérisé en ce que** l'élastomère thermoplastique est appliqué avec une température supérieure à 100°C, de préférence supérieure à 150°C, sous forme de courbes (16, 17) sur la bande porteuse (9, 11).

12. Procédé de fabrication selon la revendication 10 ou 11, **caractérisé en ce que** des premières courbes (16) d'un élastomère thermoplastique sont appliquées sur un premier support (9) et des secondes courbes (17) d'un élastomère thermoplastique sur un second support (11) et ensuite les deux supports (9, 11) sont comprimés ensemble et de ce fait les premières courbes (16) sont reliées les unes aux autres avec les autres courbes (17).
